# EUROPEAN PATENT APPLICATION

(11) **EP 4 656 631 A1**
(43) Date of publication of application: **03.12.2025**
(21) Application number: 25716964.9
(22) Date of filing: 26.02.2025
(51) Int. Cl.: C07D 307/68, C08G 63/672

(54) **2,5-FURANDICARBOXYLIC ACID PARTICLES, PREPARATION METHOD THEREFOR, AND USE THEREOF**

(30) Priority: 16.04.2024 CN 202410455919
(71) Applicant: Hefei Leaf Biotech Co., Ltd., Hefei City Anhui 230088 (CN)
(72) Inventor: XU, Hai, Hefei, Anhui 230088 (CN); CHEN, Gang, Hefei, Anhui 230088 (CN); YU, Sanxi, Hefei, Anhui 230088 (CN); XU, Qiang, Hefei, Anhui 230088 (CN)
(74) Representative: Murgitroyd & Company
(86) International application number: PCT/CN2025/079221
(87) International publication number: WO 2025/218355

(57) **Abstract**

The present application discloses 2,5-Furandicarboxylic Acid particles and a preparation method and application thereof, belonging to the technical field of polymerization reaction bio-based monomer preparation. Using 2,5-Furandicarboxylic Acid as raw material, after high-temperature dissolution in water, cooling, filtration, and drying, the FDCA particles exhibit an almost non-angular appearance, with smooth and rounded surface, high sphericity, good dispersibility, average particle diameter of 50-200µm, loose bulk density of 0.7-1.0g/mL, and repose angle of 25-40°. The present application improves the flowability of FDCA slurry by modifying the microscopic morphology and particle size distribution of FDCA solid particles, which facilitates the uniformity of materials during the polymerization process and shortens the polymerization reaction time. Additionally, it reduces the equivalent usage of ethylene glycol and helps lower the impurity content of diethylene glycol.

## Description

### TECHNICAL FIELD

The present application belongs to the technical field of bio-based monomer preparation for polymerization reactions, specifically relating to 2,5-furandicarboxylic acid particles and a preparation method and application thereof.

### BACKGROUND

2,5-Furandicarboxylic Acid (FDCA) can be produced from biomass raw materials. The typical preparation process involves biomass containing hexose sugars first undergoing dehydration to form 5-hydroxymethylfurfural (5-HMF), followed by oxidation of 5-HMF to produce FDCA. FDCA can replace petroleum-based monomer terephthalic acid (PTA) to produce high-performance polymers, such as polyethylene 2,5-furandicarboxylate (PEF), which has attracted widespread attention. Compared to polyethylene terephthalate (PET), it has better oxygen and carbon dioxide barrier properties, as well as a lower carbon footprint. Due to its biomass origin, the application of PEF plastics can reduce dependence on petroleum-based polymers.

Currently, the technology for preparing PTA by oxidation of para-xylene (PX) and the technology for producing PET by polymerization of PTA with ethylene glycol are already very mature. The global production capacity of PTA has exceeded 95 million tons, and the bottle-grade PET capacity exceeds 35 million tons. Typically in the polymerization reaction process for PET production, terephthalic acid and ethylene glycol are mixed and added to the reaction system in slurry form; In this process, to improve the uniformity of the reaction, it is preferable that the terephthalic acid slurry has good flowability, and good powder flowability is also beneficial for the handling processes of terephthalic acid such as transportation, storage, etc.; In industrial settings, to achieve good slurry properties and reaction uniformity, the excess of ethylene glycol relative to the stoichiometric amount of terephthalic acid can be adjusted, but excess ethylene glycol in the polycondensation reaction leads to increased impurities and energy consumption.

FDCA, as a substitute for PTA, similarly needs to undergo a comparable polymerization process. The slurry formed after mixing FDCA particles with ethylene glycol needs to ensure good fluidity, which is beneficial for reaction uniformity and can shorten the polymerization reaction time. Patent document CN114929679A discloses a process for producing a carboxylic acid composition including 2,5-furandicarboxylic acid, wherein the thermal treatment method involves dissolving a certain percentage of FDCA in the treatment solvent composition during heat treatment, while the remaining FDCA is maintained as a solid precipitate. The chemical equilibrium and exchange between dissolved FDCA and precipitated FDCA during the heat treatment leads to particularly advantageous particle shapes of FDCA, increased particle strength and/or beneficial particle size distribution; however, the chemical equilibrium and exchange between dissolved FDCA and precipitated FDCA is difficult to control, resulting in the problem of low FDCA particle yield. Patent document CN116120264A discloses a method for regulating the crystal particle size of 2,5-furandicarboxylic acid, wherein the method involves adding crystallization aids to the raw materials of FDCA, and by adjusting the type and content of the aids, combined with the adjustment of process parameters such as crystallization temperature, FDCA crystals with a certain particle size range can be obtained, wherein the average particle size d ₅₀ of the FDCA crystals is 20 -2000 µm, however, this method requires the use of additives, which reduces the crystallization purity and the method is relatively complex.

The flow properties of FDCA slurry are influenced by the particle size distribution and average particle size of FDCA particles. Generally, a wide particle size distribution ranging from large to small diameters tends to improve the slurry characteristics of particles. The optimal average particle diameter typically falls within the range of 50 - 150 µm. If the proportion of particles with diameters above 250 µm (40 mesh) increases, FDCA is prone to incomplete reaction during direct polymerization reactions, leading to problems such as extended reaction times and increased by-product formation. Similar to the direct polymerization method for PET, to achieve good slurry characteristics and reaction homogeneity, ethylene glycol can be used in stoichiometric excess relative to FDCA. However, excessive ethylene glycol causes problems such as increased diethylene glycol segment impurities, reduced degree of polymerization, and color deepening. However, when the amount of ethylene glycol approaches the stoichiometric ratio of FDCA, it causes the polymerization reaction system to become very viscous, resulting in uneven dispersion of solid particles, while also increasing the power consumption required for stirring.

Based on this, in order to improve the slurry characteristics during the polymerization reaction, there is an urgent need for a type of 2,5-furandicarboxylic acid particles and their preparation method to solve the aforementioned problems.

### SUMMARY

The first purpose of the present application is to provide 2,5-furandicarboxylic acid particles to solve the problems of uneven distribution and poor discreteness of existing FDCA particles.

The second purpose of the present application is to provide a preparation method for 2,5-furandicarboxylic acid particles.

The third purpose of the present application is to provide an application of 2,5-furandicarboxylic acid particles in the synthesis process of poly (ethylene 2,5-furandicarboxylate) (PEF), to solve the problems in the existing PEF preparation process, where the FDCA and ethylene glycol slurry mixture has poor flowability and homogeneity, resulting in low polymerization reaction efficiency and high impurity content of diethylene glycol.

The purposes of the present application can be achieved through the following technical solutions:
In a first aspect, a type of 2,5-furandicarboxylic acid particles, characterized in that an average particle size is 50-200µm, a loose bulk density is 0.7-1.0g/mL, and an repose angle is 25-40°.

In a second aspect, a preparation method for 2,5-furandicarboxylic acid particles, comprising the following steps:
adding 2,5-furandicarboxylic acid raw material into a high-pressure reactor, introducing deionized water into the high-pressure reactor to obtain a mixed liquid, stirring and heating, allowing the system temperature to rise from room temperature to 100-150°C, then maintaining the temperature for reaction; after the heat preservation, cooling down to room temperature; filtering and collecting the material, then washing to obtain wet 2,5-furandicarboxylic acid, which is then dried to produce 2,5-furandicarboxylic acid particles.

As a further embodiment of the present application, the purity of the 2,5-furandicarboxylic acid raw material is >95%.

As a further embodiment of the present application, the pressure of the system in the high-pressure reactor < 1.5 MPa.

As a further embodiment of the present application, the mass percentage of 2,5-furandicarboxylic acid in the mixed solution is 5%-20%.

As a further embodiment of the present application, the stirring rate is 200-400rpm.

As a further embodiment of the present application, the heating time is 2-4h.

As a further embodiment of the present application, the duration of the heat preservation is 0.5-6h.

As a further embodiment of the present application, the cooling time is 2-10h.

In a third aspect, an application of 2,5-furandicarboxylic acid particles prepared by the preparation method for 2,5-Furandicarboxylic Acid particles in polymerization reaction.

Compared with the existing technology, the beneficial effects of the present application are:
1. The present application discloses a 2,5-Furandicarboxylic Acid with a relatively simple preparation method. The FDCA particles obtained through this preparation method have almost no angular features at the microscopic level, with smooth and rounded particle surfaces, high sphericity, good discreteness, high bulk density, an average particle size of 50-200µm, a loose packing density of 0.7-1.0g/mL, and a repose angle of 25-40°.
2. The present application significantly improves the flowability of FDCA slurry by altering the appearance and particle size distribution of FDCA solid particles, which is beneficial for enhancing the homogeneity of polymerization reaction materials while shortening the polymerization reaction time. During the polymerization process of PEF, it can reduce the required equivalents of Ethylene Glycol, which helps to lower the content of Diethylene Glycol impurities.

### BRIEF DESCRIPTION OF THE DRAWINGS

The following provides further explanation of the present application in conjunction with the figures.
Figure 1 is a microscopic morphology image of FDCA particles prepared in embodiment 1 of the present application;
Figure 2 is a microscopic morphology image of the FDCA raw material in the present application.

### DETAILED DESCRIPTION

The following will combine the embodiments of the present application to provide a clear and complete description of the technical solutions in the embodiments of the present application. Obviously, the described embodiments are only a portion of the embodiments of the present application, not all the embodiments. Based on the embodiments in the present application, all other embodiments obtained by those skilled in the art without creative effort fall within the protection scope of the present application.

A preparation method for 2,5-furandicarboxylic acid particles, comprising the following steps:
adding 2,5-furandicarboxylic acid raw material with purity >95% into a high-pressure reactor, adding deionized water into the high-pressure reactor to obtain a mixed solution, wherein the mass percentage of 2,5-furandicarboxylic acid in the mixed solution is 5%-20%, stirring and heating, with a stirring rate of 200-400rpm, so that the system temperature rises from room temperature to 100-150°C, with a heating time of 2-4 h, then maintaining the temperature for reaction for 0.5-6h, during the reaction process the pressure of the system in the high-pressure reactor is <1.5 MPa; after the temperature maintenance is completed, cooling down to room temperature, with a cooling time of 2-10h; filtering and collecting the material, then washing to obtain wet 2,5-furandicarboxylic acid, which is then dried to produce 2,5-furandicarboxylic acid particles.

### Embodiment 1

A preparation method for 2,5-furandicarboxylic acid particles, comprising the following steps:
adding 100g of 2,5-furandicarboxylic acid raw material with a purity of 99.5% into a high-pressure reactor, then adding 400g of deionized water to the high-pressure reactor to obtain a mixed solution. After sealing, start stirring and heating with a stirring rate of 300rpm, raising the system temperature from room temperature to 145°C over a period of 2.5h, then maintaining the temperature for a reaction time of 6h. During the reaction process, the pressure in the high-pressure reactor system is 1 MPa; after the heating period is complete, cooling down to room temperature over a period of 4h; filtering and collecting the material, rinsing with a small amount of deionized water to obtain wet product 2,5-furandicarboxylic acid, and after drying, obtaining 2,5-furandicarboxylic acid particles.

### Embodiment 2

A preparation method for 2,5-furandicarboxylic acid particles, wherein the preparation method is the same as Embodiment 1, except that 1900g of deionized water is added to the high-pressure reactor, with all other components and parameters remaining unchanged.

### Embodiment 3

A preparation method for 2,5-furandicarboxylic acid particles, wherein the preparation method is the same as Embodiment 1, except that 990g of deionized water is added to the high-pressure reactor, with all other components and parameters remaining unchanged.

### Embodiment 4

A preparation method for 2,5-furandicarboxylic acid particles, wherein the preparation method is the same as Embodiment 1, except that the duration of the heat preservation after temperature increase is 1.5h, with all other components and parameters remaining unchanged.

### Embodiment 5

A preparation method for 2,5-furandicarboxylic acid particles, wherein the preparation method is the same as Embodiment 1, except that the duration of the heat preservation after temperature increase is 5.5h, while other components and parameters remain unchanged.

### Embodiment 6

A preparation method for 2,5-furandicarboxylic acid particles, wherein the preparation method is the same as Embodiment 1, except that the system temperature rises from room temperature to 110°C, with a heating time of 2h, while other components and parameters remain unchanged.

### Embodiment 7

A preparation method for 2,5-furandicarboxylic acid particles, wherein the preparation method is the same as Embodiment 1, except that the system temperature rises from room temperature to 130°C, with a heating time of 2h, while other components and parameters remain unchanged.

### Embodiment 8

A preparation method for 2,5-furandicarboxylic acid particles, wherein the preparation method is the same as Embodiment 1, except that after the heat preservation is completed, the mixture is cooled down to room temperature, with a cooling time of 1.5h, while other components and parameters remain unchanged.

### Embodiment 9

A preparation method for 2,5-furandicarboxylic acid particles, wherein the preparation method is the same as Embodiment 1, except that after the heat preservation is completed, the mixture is cooled down to room temperature, with a cooling time of 6h, while other components and parameters remain unchanged.

### Embodiment 10

A preparation method for 2,5-furandicarboxylic acid particles, wherein the preparation method is the same as Embodiment 1, except that after the heat preservation is completed, the mixture is cooled down to room temperature, with a cooling time of 9h, while other components and parameters remain unchanged.

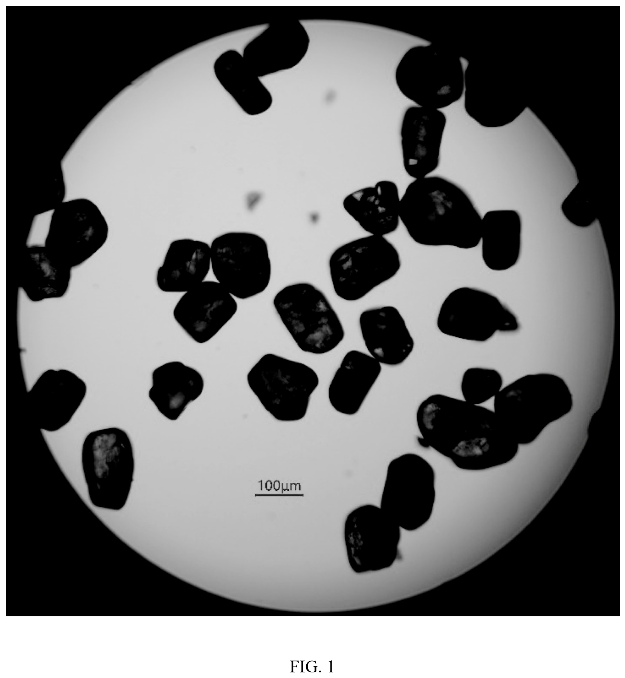

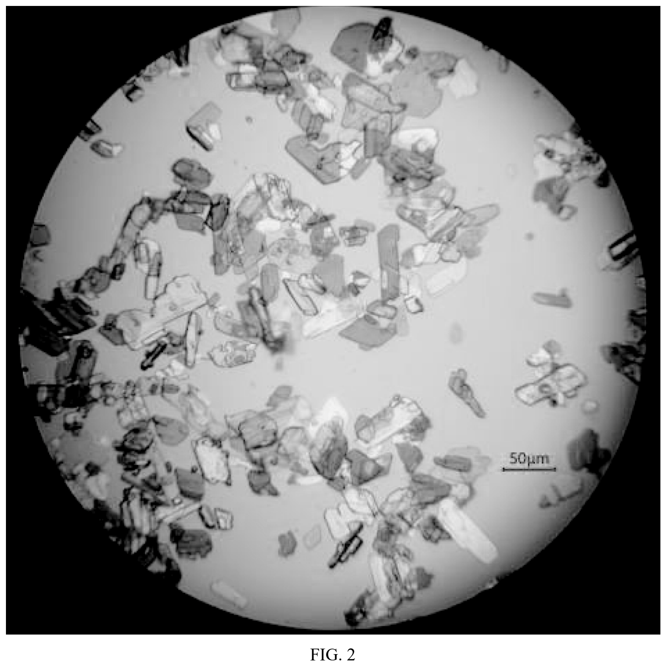

Performance characterization of 2,5-furandicarboxylic acid particles prepared in embodiments 1-10:
(1) Microscopic morphology characterization: Figure 1 shows the microscopic morphology of 2,5-furandicarboxylic acid particles prepared in embodiment 1, and Figure 2 shows the microscopic morphology of 2,5-furandicarboxylic acid raw material; By comparing Figures 1 and 2, it can be observed that the appearance of FDCA particles after reaction has almost no angular edges, the particle surface is round and smooth, with high sphericity and good flowability;
(2) Average particle size: calculated after screening through standard sieves;
(3) Bulk density: The bulk density of the particles is determined using the test method specified in GB/T 16913-2008;
(4) Repose angle test: The repose angle of the particles is determined using the test method specified in GB/T 16913-2008;

The test results are shown in Table 1:/

**Table 1**

| Implementation Examples | Average Particle Size (µm) | Bulk Density (g/mL) | Repose Angle |
|---|---|---|---|
| Embodiment 1 | 85µm | 0.98g/mL | 25° |
| Embodiment 2 | 195µm | 0.80g/mL | 38° |
| Embodiment 3 | 136µm | 0.78g/mL | 36° |
| Embodiment 4 | 154µm | 0.83g/mL | 35° |
| Embodiment 5 | 110µm | 0.99g/mL | 28° |
| Embodiment 6 | 55µm | 0.74g/mL | 39° |
| Embodiment 7 | 75µm | 0.83g/mL | 36° |
| Embodiment 8 | 1366µm | 0.73g/mL | 37° |
| Embodiment 9 | 84µm | 0.98g/mL | 30° |
| Embodiment 10 | 65µm | 0.99g/mL | 32° |

As can be seen from Table 1, the mass fraction of FDCA in the FDCA-water mixture, heating temperature and holding time, and cooling time are all factors affecting the average particle size, bulk density, and repose angle of FDCA particles. Optimizing reaction conditions can yield FDCA particles of better quality.

### Embodiment 11

An application method of 2,5-furandicarboxylic acid particles, using the synthesis of polyethylene furanoate as a model example of polymerization reaction, comprising the following steps:
adding 2,5-furandicarboxylic acid particles (15.6g, 0.1mol) prepared in Embodiment 1 and ethylene glycol (6.8g, 0.11mol) to the slurry tank, thoroughly mixing, then transferring to the esterification vessel, reacting at 190°C for 2h, then stopping the reaction and discharging water; adding catalyst germanium oxide 0.78g to the dimethyl furandicarboxylate generated in the esterification step, then performing melt polycondensation in the polycondensation reactor, reacting at 70pa pressure and 235°C for 3h, then stopping the reaction to obtain the polymer PEF (polyethylene furanoate).

### Comparative Example 1

Adding 2,5-Furandicarboxylic Acid raw material with a purity of 99.5% (15.6g, 0.1mol) and Ethylene Glycol (6.8g, 0.11mol) to the slurry tank, thoroughly mixing, and then transferring to the esterification vessel, reacting at 190°C for 2h, then stopping the reaction and discharging the water; adding catalyst germanium oxide 0.78g to the dimethyl furandicarboxylate generated in the esterification step, then performing melt polycondensation in the polycondensation reactor, reacting at 70pa pressure and 235°C for 3h, then stopping the reaction to obtain the polymer PEF (polyethylene furanoate).

Performance testing of PEF polyesters prepared in Embodiment 11 and Comparative Example 1:
Diethylene Glycol content test: The methanol ester exchange method in GB/T 14190-2017 was used to test the Diethylene Glycol content;
PEF polyester molecular weight test: Gel Permeation Chromatography (GPC) was used to determine the polyester molecular weight;
The test results are shown in Table 2;

**Table 2**

| Implementation Examples | Diethylene Glycol Content | PEF Molecular Weight |
|---|---|---|
| Embodiment 11 | 1.35% | 31000 |
| Comparative Example 1 | 1.54% | 18000 |

As shown in Table 2, when preparing PEF under the same parameter conditions, the higher quality FDCA particles obtained through optimized reaction conditions are beneficial for improving the uniformity of materials during the PEF polymerization process. This also helps to shorten the polymerization reaction time. More importantly, it can reduce the equivalent usage of ethylene glycol, thereby helping to lower the content of impurity diethylene glycol.

It should be noted that in this document, relational terms such as 'first' and 'second' are only used to distinguish one entity or operation from another entity or operation, and do not necessarily require or imply any actual relationship or sequence between these entities or operations. Moreover, the terms "comprise," "contain," or any other variants thereof are intended to cover non-exclusive inclusion, such that a process, method, article, or device that includes a series of elements not only includes those elements but also includes other elements not explicitly listed, or elements inherent to such process, method, article, or device.

Although the embodiments of the present application have been shown and described, it will be understood by those skilled in the art that various changes, modifications, substitutions, and variations can be made to these embodiments without departing from the principles and spirit of the present application, the scope of which is defined by the appended claims and their equivalents.

## Claims

1. A 2,5-Furandicarboxylic Acid particle, **characterized in that** an average particle size is 50-200µm, a loose packing density is 0.7-1.0g/mL, and a repose angle is 25-40°.

2. A preparation method for the 2,5-Furandicarboxylic Acid particles according to claim 1, **characterized in that** comprising the following steps:
adding 2,5-furandicarboxylic acid raw material into a high-pressure reactor, introducing deionized water into the high-pressure reactor to obtain a mixed liquid, stirring and heating, allowing the system temperature to rise from room temperature to 100-150°C, then maintaining the temperature for reaction; after the heat preservation, cooling down to room temperature; filtering and collecting the material, then washing to obtain wet 2,5-furandicarboxylic acid, which is then dried to produce 2,5-furandicarboxylic acid particles.

3. The preparation method for 2,5-furandicarboxylic acid particles according to claim 2, **characterized in that** the purity of the 2,5-furandicarboxylic acid raw material is >95%.

4. The preparation method for 2,5-Furandicarboxylic Acid particles according to claim 2, **characterized in that** the pressure in the high-pressure reactor system < 1.5 MPa.

5. The preparation method for 2,5-Furandicarboxylic Acid particles according to claim 2, **characterized in that** the mass percentage of 2,5-Furandicarboxylic Acid in the mixed liquid is 5%-20%.

6. The preparation method for 2,5-Furandicarboxylic Acid particles according to claim 2, **characterized in that** the stirring rate is 200-400 rpm.

7. The preparation method for 2,5-furandicarboxylic acid particles according to claim 2, **characterized in that** the heating time is 2-4 hours.

8. The preparation method for 2,5-furandicarboxylic acid particles according to claim 2, **characterized in that** the duration of the heat preservation is 0.5-6h.

9. The preparation method for 2,5-furandicarboxylic acid particles according to claim 2, **characterized in that** the cooling time is 2-10h.

10. An application of the 2,5-furandicarboxylic acid particles according to claim 1 in polymerization reactions.
